# FASCICULE DE BREVET EUROPEEN

(11) **EP 0 925 273 B1**
(45) Date de publication et mention de la délivrance du brevet: **07.11.2001**
(21) Numéro de dépôt: 97936682.0
(22) Date de dépôt: 05.08.1997
(51) Int. Cl.: C07C 59/84, A61K 31/19, C07D 307/77, C07D 317/60, C07C 59/86, C07C 59/88, C07C 59/90

(54) **COMPOSITIONS PHARMACEUTIQUES CONTENANT DES ACIDES 4-OXO-BUTANOIQUES**
A-OXO-BUTANSÄUREN ENTHALTENDE PHARMAZEUTISCHE ZUSAMMENSETZUNG
PHARMACEUTICAL COMPOSITION CONTAINING 4-OXO-BUTYNIC ACIDS

(30) Priorité: 16.08.1996 FR 9610254
(43) Date de publication de la demande: 30.06.1999
(73) Titulaire: MERCK PATENT GmbH, 64293 Darmstadt (DE)
(72) Inventeur: MOINET, Gérard, F-91400 Orsay (FR); DOARE, Lilian, F-91170 Viry Châtillon (FR); KERGOAT, Micheline, F-91440 Bures sur Yvette (FR); MAIZERAY, Philippe, F-92170 Vanves (FR); MESANGEAU, Didier, F-77380 Combs la Ville (FR)
(86) Numéro de dépôt international: EP9704252
(87) Numéro de publication internationale: WO9807681

(56) Documents cités:
- FR-A- 2 623 507

## Description

La présente invention concerne des compositions pharmaceutiques contenant des acides 4-oxo-butanoïques, qui sont utiles notamment dans le traitement du diabète.

La présente invention a ainsi pour objet des compositions pharmaceutiques comprenant, à titre de principe actif, un composé de formule : 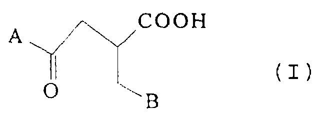
dans laquelle les groupes A et B sont choisis indépendamment l'un de l'autre parmi :
   - un groupe aryle mono-, bi- ou tricyclique ayant de 6 à 14 atomes de carbone;
   - un groupe hétéroaromatique choisi parmi les groupes pyridyle, pyrimidyle, pyrrolyle, furyle et thiényle;
   - un groupe alcoyle ayant de 1 à 14 atomes de carbone;
   - un groupe cycloalcoyle ayant de 5 à 8 atomes de carbone;
   - un groupe hétérocyclique saturé choisi parmi les groupes tétrahydrofuryle, tétrahydropyrranyle, pipéridinyle et pyrrolidinyle;
les groupes A et B pouvant porter 1 à 3 substituants choisis parmi un groupe alcoyle en C₁-C₆, alcoxy en C₁-C₆, aryle en C₆-C₁₄, hétéroaryle choisi parmi pyridyle, pyrimidyle, pyrrolyle, furyle et thiényte, aryl (C₆-C₁₄) alcoyle (C₁-C₆), aryl (C₆-C₁₄) alcoyl (C₁-C₆) aryle (C₆-C₁₄), un halogène, un groupe trifluorométhyle, trifiuorométhoxy, cyano, hydroxy, nitro, amino, carboxy, alcoxy (C₁-C₆)carbonyle, carbamoyle, alcoyl(C₁-C₆)sulfonyle, sulfoamino, alcoyl(C₁-C₆) sulfonylamino, sulfamoyle, alcoyl(C₁-C₆)carbonylamino
ou deux des substituants formant un groupe methylènedioxy, son solvate ou un sel de cet acide avec une base pharmaceutiquement acceptable.

Dans une forme préférée de réalisation de l'invention, les compositions comprennent à titre de principes actifs, un composé de formule I dans laquelle A et B sont choisis parmi des groupes aryle.

Comme exemple de groupes aryle, on peut citer les groupes phényle, α-naphtyle, β-naphtyle, fluorényle.

Les groupes alcoyle en C₁-C₆ peuvent être linéaires ou ramifiés. Comme exemples on peut citer les groupes méthyle, éthyle, propyle, isopropyle, butyle, isobutyle, tertiobutyle, pentyle.

Les groupes alcoxy en C₁-C₆ peuvent de même être linéaires ou ramifiés.

Comme exemples on peut citer les groupes méthoxy, éthoxy, propoxy, isopropoxy, butoxy, isobutoxy.

Les halogènes peuvent être choisis parmi le fluor, le chlore, le brome et l'iode.

La présente invention englobe les compositions qui contiennent les formes tautomères, les énantiomères, les diastéréoisomères et les épimères des composés de formule I.

Comme exemples de sels pharmaceutiquement acceptables, on peut citer les sels de sodium; sels de potassium, les sels de magnésium, les sels de calcium, les sels d'amine et autres sels du même type (aluminium, fer, bismuth, etc ...)

Dans un mode de réalisation préféré, les compositions selon l'invention comprennent un composé choisi parmi:
- l'acide 2-benzyl-4-(4-méthoxyphényl)-4-oxobutanoïque
- l'acide 2-benzyl-4-(4-fluorophényl)-4-oxobutanoïque
- l'acide 2-cyclohexylméthyl-4-(4-méthoxyphényl)-4-oxobutanoïque
- l'acide 2-benzyl-4-phényl-4-oxobutanoïque
- l'acide 2-(β-naphthylméthyl)-4-phényl-4-oxobutanoïque
- l'acide 2-benzyl-4-(β-naphtyl)-4-oxobutanoïque
- l'acide 2-[(4-chlorophényl)méthyl]-4-(4-méthoxyphényl)-4-oxobutanoïque
- l'acide 2-benzyl-4-(4-méthylphényl)-4-oxobutanoïque
- l'acide 4-(4-fluorophényl)-2-[(4-méthoxyphényl)méthyl]-4-oxobutanoïque
- l'acide 2-benzyl-4-(3,4-méthylènedioxyphényl)-4-oxobutanoïque
- l'acide 2-benzyl-4-cyclohexyl-4-oxobutanoïque
- l'acide 4-phényl-2-[(tétrahydrofur-2-yl)méthyl]-4-oxobutanoïque,
les solvates et les sels de ces acides avec des bases pharmaceutiquement acceptables.

Certains composés de formule I sont connus (Bioorg. Chem. 14, 148, 1986; Biochemistry 23, 2083, 1984; J.A.C.S. 100, 7750, 1978; EP-A-310918 et DE-A-3 839 401).

La présente invention a également pour objet les composés nouveaux de formule I, c'est-à-dire les composés de formule : 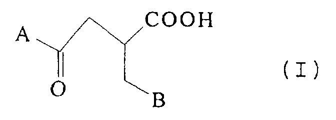
dans laquelle les groupes A et B sont choisis indépendamment l'un de l'autre parmi :
   - un groupe aryle mono-, bi- ou tricyclique ayant de 6 à 14 atomes de carbone;
   - un groupe hétéroaromatique choisi parmi les groupes pyridyle, pyrimidyle, pyrrolyle, furyle et thiényle;
   - un groupe alcoyle ayant de 1 à 14 atomes de carbone;
   - un groupe cycloalcoyle ayant de 5 à 8 atomes de carbone;
   - un groupe hétérocyclique saturé choisi parmi les groupes tétrahydrofuryle, tétrahydropyrranyle, pipéridinyle et pyrrolidinyle;
les groupes A et B pouvant porter 1 à 3 substituants choisis parmi un groupe alcoyle en C₁-C₆, alcoxy en C₁-C₆, aryle en C₆-C₁₄, hétéroaryle choisi parmi pyridyle, pyrimidyle, pyrroiyle, furyle et thiényle, aryl (C₆-C₁₄) alcoyle (C₁-C₆), aryl (C₆-C₁₄) alcoyl (C₁-C₆) aryle (C₆-C₁₄), un halogène, un groupe trifluorométhyle, trifluorométhoxy, cyano, hydroxy, nitro, amino, carboxy, alcoxy (C₁-C₆)carbonyle, carbamoyle, alcoyl(C₁-C₆)sulfonyle, sulfoamino, alcoyl(C₁-C₆) sulfonylamino, sulfamoyle, alcoyl(C₁-C₆)carbonylamino
ou deux des substituants formant un groupe methylènedioxy,
à l'exclusion des composés de formule I dans laquelle B est un groupe phényle non substitué et A est un groupe phényle, 4-méthoxyphényle, 4-chlorophényle ou cyclohexyle,
leurs solvates et les sels de ces acides avec des bases.

Les composés nouveaux incluent les sels des acides avec des bases pharmaceutiquement acceptables ou d'autres bases qui donnent des sels pouvant servir à l'identification, à la purification ou au dédoublement des composés de formule I.

Les composés de formule I peuvent êtrepréparés selon une synthèse malonique qui consiste à faire réagir un composé de formule : 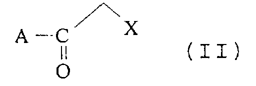
dans laquelle X est un halogène comme défini précédemment, et A a la signification donnée précédemment,
avec un dérivé maionique de formule : 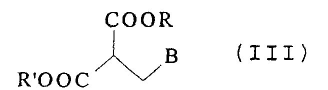
dans laquelle R et R' sont des groupes alcoyle en C₁-C₆ et B a la signification donnée précédemment,
en présence d'un hydrure de métal alcalin ou d'un aicoolate de métal alcalin, pour former un composé de formule : 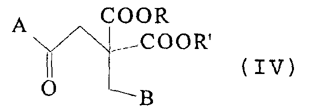
dans laquelle A, B, R et R' ont la signification donnée précédemment, puis à saponifier le composé de formule IV, par exemple par un mélange d'hydroxyde alcalin, d'eau, de tétrahydrofuranne et/ou d'éthanol, pour former un composé de formule : 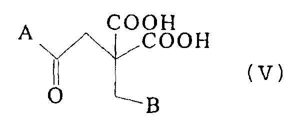
puis à décarboxyler le composé de formule V, notamment par chauffage à sec, pour donner le composé de formule I.

La séparation des énantiomères des composé de formule (I) peut être effectuée par recristallisation successive du sel de l'acide (I) avec une base optiquement active dans des solvants tels que l'acétone, l'acétate d'éthyle, l'isopropanol,... puis déplacement du sel en acide optiquement actif par un acide minéral ou organique selon une méthode classique.

Les compositions selon la présente invention peuvent être utilisées dans le traitement du diabète, notamment du diabète non insulinodépendant en raison de leur effet hypoglycémiant et de leur absence de toxicité aux doses actives.

Les compositions pharmaceutiques selon l'invention peuvent être présentées sous des formes destinées à l'administration par voie parentérale, orale, rectale, permuqueuse ou percutanée.

Elles seront donc présentes sous forme de solutés ou de suspensions injectables ou flacons multi-doses, sous forme de comprimés nus ou enrobés, de dragées, de capsules, de gélules, de pilules, de cachets, de poudres, de suppositoires ou de capsules rectales, de solutions ou de suspensions, pour l'usage percutané dans un solvant polaire, ou pour l'usage permuqueux.

Les excipients qui conviennent pour de telles administrations sont les dérivés de la cellulose ou la cellulose microcristalline, les carbonates alcalino-terreux, le phosphate de magnésium, les amidons, les amidons modifiés, le lactose pour les formés solides.

Pour l'usage rectal, le beurre de cacao ou les stéarates de polyéthylèneglycol sont les excipients préférés.

Pour l'usage parentéral, l'eau, les solutés aqueux, le sérum physiologique, les solutés isotoniques sont les véhicules les plus commodément utilisés.

La posologie peut varier dans les limites importantes en fonction de l'indication thérapeutique et de la voie d'administration, ainsi que de l'âge et du poids du sujet.

Les exemples suivants illustrent la préparation des composés de formule I.

### Exemple 1

### Préparation de l'acide 2-benzyl-4-(4-méthoxyphényl)-4-oxobutanoïque (produit n° 2)

### A - Préparation du 2-benzyl-2-[2-4-méthoxyphényl)-2-oxoéthyl] propanedioate de diéthyle

On chauffe à 70° C un mélange de 24 ml de 2-benzylmalonate de diéthyle, 3 g d'hydrure de sodium à 80 % dans l'huile (préalablement lavé à l'éther de pétrole) et 150ml de tétrahydrofuranne pendant 1 heure. A + 5° C, on ajoute en 1 heure, 24 g de 2-bromo-4'-méthoxyacétophénone en solution dans 50 ml de tétrahydrofuranne. Après une nuit à température ambiante, on verse le mélange réactionnel dans 400 ml d'eau. Après extraction à l'acétate d'éthyle, la solution organique est lavée à la saumure, séchée sur sulfate de magnésium et est concentrée à sec sous pression réduite. On obtient 40 g d'une huile jaune qui cristallise.
F = 67° C (hexane)
I.R. (KBr) υ CO (cétone) = 1671 cm⁻¹; υ CO (ester): 1735 cm⁻¹
RMN^{1H} (DMSO/TMS)
1,2 (6H, t, 2CH₃); 3,35 (4H, d, 2CH₂); 3,8 (3H, s, OCH₃); 4,1 (4H, q, 2OCH₂); 7,1 (7H, m, H aromatique); 7,85 (2H, d, H aromatique).

### B - Préparation de l'acide 2-benzyl-2-[2-(4-méthoxyphényl)-2-oxoéthyl]propanedioïque

Sous vive agitation, on mélange 30 g du 2-benzyl-2-[2-(4-méthoxyphényl)-2-oxoéthyl]propanedioate de diéthyle, 80 ml de soude aqueuse 2N et 250 ml de tétrahydrofuranne.

Après 5 jours à température ambiante, le mélange réactionnel est versé sur 1 litre d'eau glacée. Le mélange est lavé deux fois avec 200 ml d'acétate d'éthyle puis est acidifié à froid avec 60 ml d'acide chlorhydrique aqueux 3N.

On extrait avec trois fois 200ml d'acétate d'éthyle. La phase organique est lavée avec 100ml d'eau neutre, 100 ml de saumure, puis séchée sur sulfate de magnésium et enfin concentrée à sec sous pression réduite. Le solide jaune gras est recristallisé dans de l'acétonitrile. On obtient 18 g d'un solide blanc.
F = 175° C, décomp.
I.R. (KBr) υ CO (cétone) = 1660 cm⁻¹; υ CO (acide) = 1749 cm⁻¹
RMN^{1H} (DMSO/TMS)
3,3 (4H, s, 2CH₂); 3,8 (3H, s, OCH₃); 7 (7H, m, H aromatique); 7,8 (2H, d, H aromatique); 13,7 (m, OH).

### C - Préparation de l'acide 2-benzyl-4-(4-méthoxyphényl)-4-oxobutanoïque

17 g d'acide 2-benzyl-2-[2-(4-méthoxyphényl)-2-oxoéthyl]propanedioïque sont chauffés sous agitation jusqu'à fusion. Lorsque le dégagement gazeux cesse, on arrête le chauffage et refroidit à température ambiante. Le solide jaune est recristallisé dans de l'acétate d'éthyle. On obtient 12 g d'un solide blanc.
F = 131° C
I.R. (KBr) υ CO (cétone) = 1664 cm⁻¹; υ CO (acide) = 1729 cm⁻¹
RMN^{1H} (DMSO/TMS)
3,1 (5H, m, CH et 2CH₂); 3,8 (3H, s, OCH₃); 7,1 (7H, m, H aromatique); 7,9 (2H, d, H aromatique); 13,5 (m, OH).

### EXEMPLE 2

### Préparation de l'acide (-)-2-benzyl-4-(4-méthoxyphényl)-4-oxobutanoïque (produit n° 3)

20 g d'acide obtenu à l'exemple 1 sont solubilisés dans 200 ml d'acétone tiède. On ajoute 8,12 g de S-(-)-α-méthylbenzylamine en solution dans 40 ml d'acétone. On refroidit le mélange, filtre le solide blanc et essore. Après recristallisation successive dans l'isopropanol jusqu'à stabilisation de la déviation optique du sel, on obtient 7,3 g d'un sel, qui est traité par 100 ml d'acide chlorhydrique 2N sous vive agitation et 50 ml d'éther éthylique. On décante et extrait une nouvelle fois la phase aqueuse acide avec 50 ml d'éther éthylique. On lave les solutions éthérées réunies une fois à l'eau neutre puis une fois à la saumure. La solution organique est ensuite séchée sur sulfate de magnésium et concentrée à sec sous pression réduite à température ambiante. L'huile résiduelle cristallise dans le pentane. On obtient 5,1 g d'un solide blanc
F = 94° C;
pureté HPLC > 95 %;
[α]²²_{D} = -18°1 (c = 5, AcOEt).

### Exemple 3

### Préparation de l'acide (+)-2-benzyl-4-(4-méthoxyphényl)-4-oxobutanoïque (produit n° 4)

La technique est la même que celle utilisée pour séparer l'acide (-) sauf qu'on utilise ici le R-(+)-α-méthylbenzylamine. On obtient un solide blanc.
F = 93° C;
HPLC = 98 %;
[α]¹⁹_{D} = + 17°6 (c = 5, AcOEt).

### Exemple 4

### Préparation de l'acide 2-benzyl-4-(4-fluorophényl)-4-oxobutanoïque (produit n° 5)

### A - Préparation du 2-benzyl-2-[2-(4-fluorophényl)-2-oxoéthyl] propanedioate d'éthyle

On procède de la même manière que dans l'exemple 1 stade A. On obtient une huile jaune cuivrée.
I.R. (film) = υ CO (cétone) = 1687 cm⁻¹; υ CO (ester) = 1735 cm⁻¹.
RMN^{1H} (DMSO/TMS) = 1,25 (6H, t, 2CH₃); 3,5 (4H, d, 2CH₂); 4,2 (4H, q, 2OCH₂); 6,8-8 (9H, m, H aromatique).

### B - Préparation de l'acide 2-benzyl-2-[2-(4-fluorophényl)-2-oxoéthyl] propanedioïque

On procède de la même manière que dans l'exemple 1 stade B. On obtient un solide blanc.
F = 185-90° C (acétonitrile)
I.R. (KBr) : υ CO (cétone) = 1675 cm⁻¹; υ CO (acide) = 1747 cm⁻¹.
RMN^{1H} (DMSO/TMS) : 3,3 (4H, d, 2CH₂); 6,7-8 (9H, m, H aromatique); 13 (m, OH).

### C - Préparation de l'acide 2-benzyl-4-(4-fluorophényl)-4-oxobutanoïque

On procède de la même manière que dans l'exemple 1 stade C. On obtient un solide jaune qui est recristallisé dans l'acétonitrile. Le solide blanc ainsi obtenu correspond à l'acide.
F = 140° C
I.R. (KBr) = υ CO (cétone) = 1683 cm ⁻¹; υ CO (acide) = 1708 cm⁻¹.
RMN^{1H} (DMSO/TMS) : 3 (5H, m, CH et 2CH₂); 7-8,1 (9H, m, H aromatique); 12,2 (s, OH).

### Exemple 5

### Préparation de l'acide (+)-2-benzyl-4-(4-fluorophényl)-4-oxobutanoïque (produit n° 6)

On utilise la même méthode que pour l'exemple 4. On obtient un solide blanc.
F = 88° C;
HPLC = 99,8 %;
[α]²²_{D} = + 9°9 (c = 5, AcOEt).

### Exemple 6

### Préparation de l'acide (-)-2-benzyl-4-(4-fluorophényl)-4-oxobutanoïque (produit n° 7)

On utilise la mêmeméthode que pour l'exemple 3. On obtient un solide blanc.
F = 89° C;
HPLC = 98,5 %;
[α]²²_{D} = - 8° 9 (c = 10, AcOEt).

On a rassemblé dans le Tableau I suivant les caractéristiques des composés de formule I.

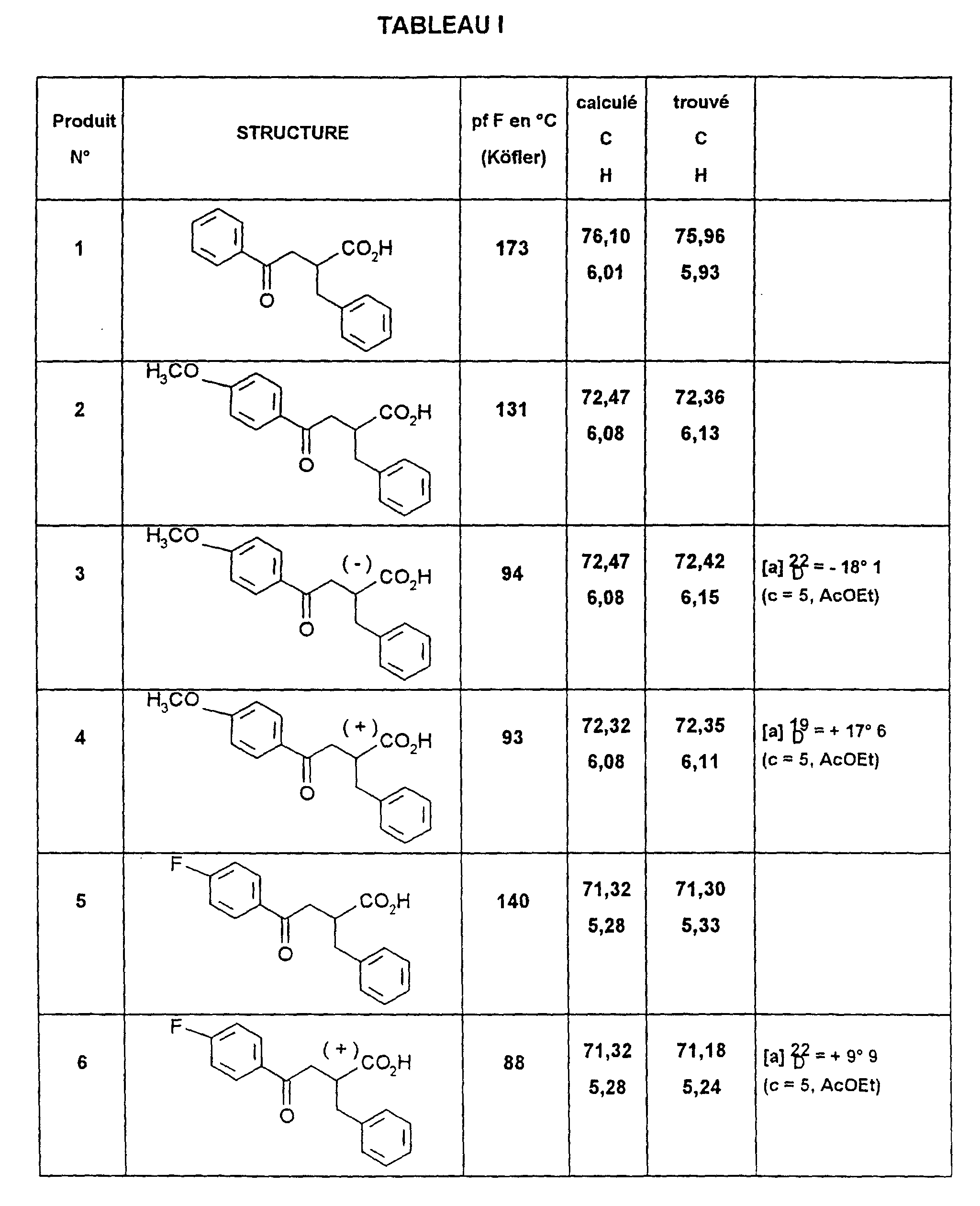

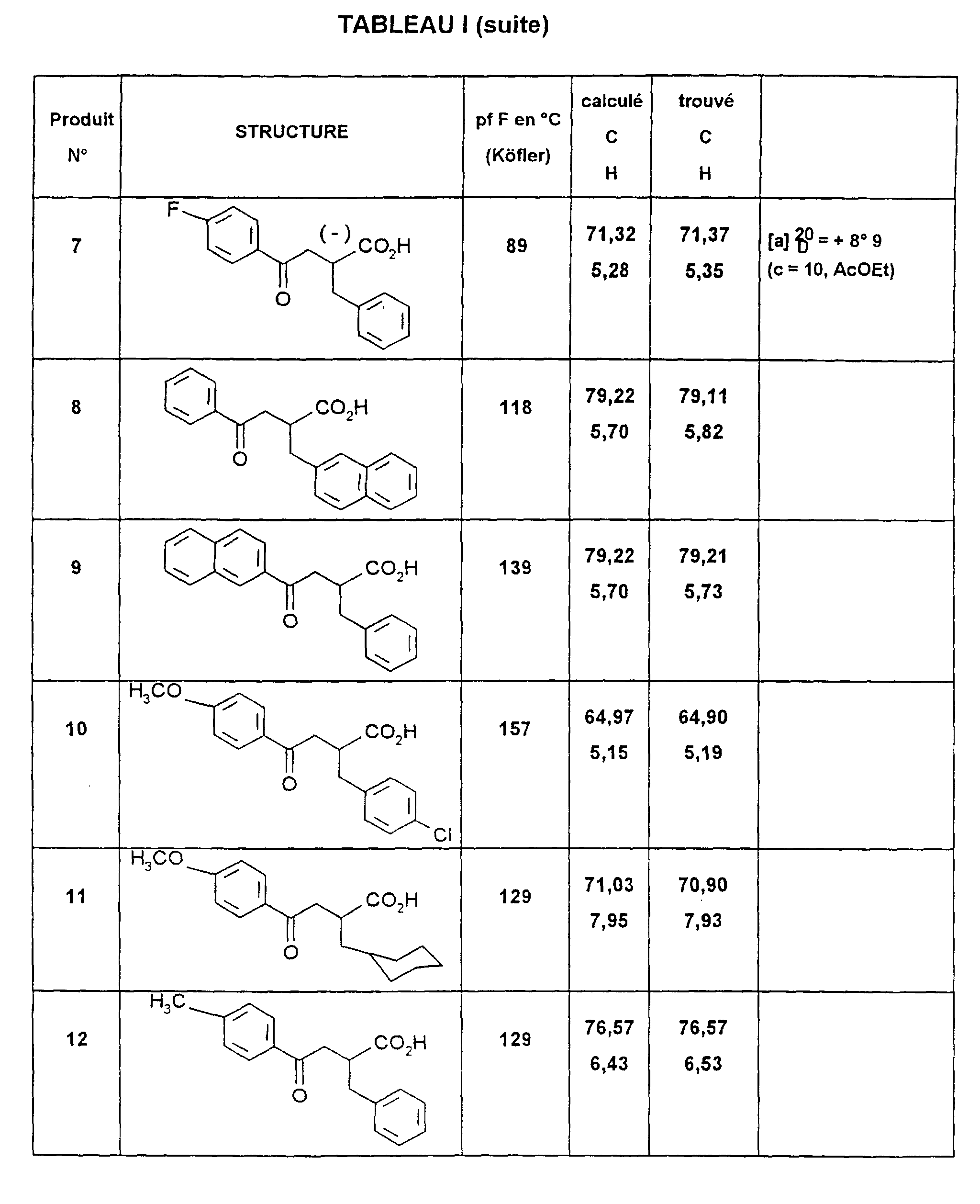

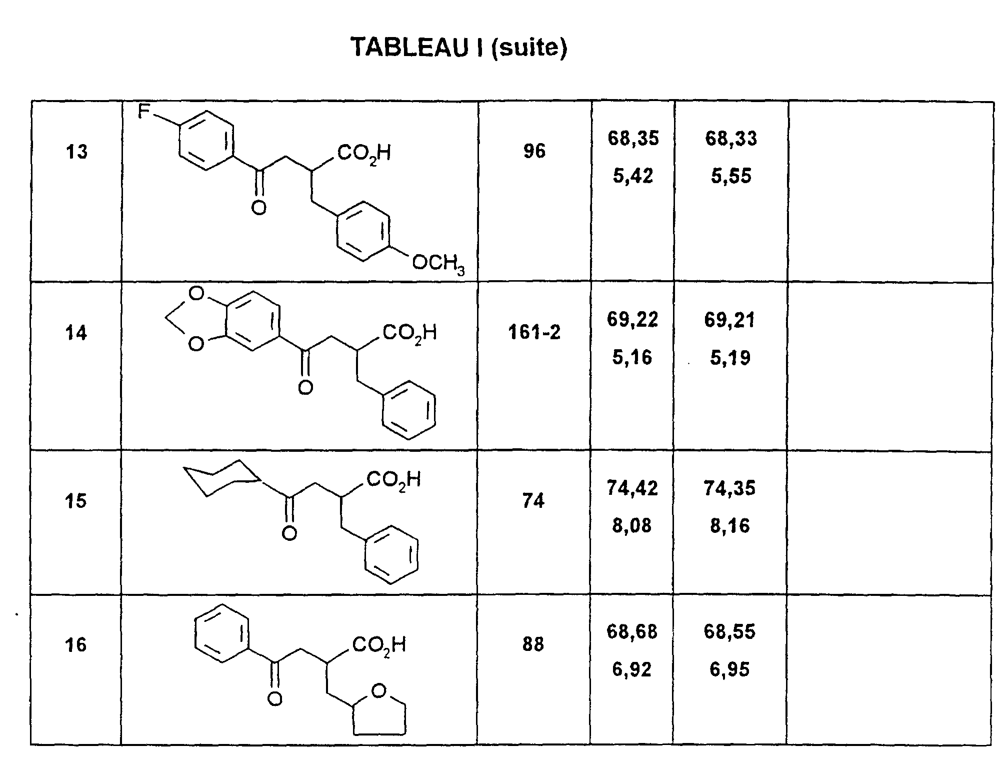

On donnera ci-après des résultats des études pharmacologiques.

### 1 - Etude de l'activité antidiabétique chez le rat nOSTZ

On a déterminé l'activité antidiabétique des composés de formule I par voie orale sur un modèle expérimental de diabète non insulinodépendant, induit chez le rat par la Streptozotocine.

Le modèle de diabète non insulinodépendant est obtenu chez le rat par une injection néonatale (le jour de la naissance) de Streptozotozine.

Les rats diabétiques utilisés sont âgés de 8 semaines.

La stabulation des animaux est réalisée, du jour de leur naissance au jour de l'expérimentation, dans une animalerie à température régulée de 21 à 22° C, et soumise à un cycle fixe de lumière (de 7 H à 19 H) et d'obscurité (de 19 H à 7 H). Leur alimentation a consisté en un régime d'entretien; eau et nourriture ont été fournies "ad libitum", à l'exception du jeûne de 2 heures précédant les tests où la nourriture est retirée (état postabsorptif).

Les rats sont traités par voie orale pendant le jour avec le produit à tester. 2 heures après la dernière administration du produit et 30 minutes après anesthésie des animaux au Pentobarbital Sodique (Nembutal®), un prélèvement sanguin de 300 *µ*l est effectué à l'extrémité de la queue.

Le Tableau II rassemble les principaux résultats obtenus.

Ces résultats montrent l'efficacité des composés de formule I pour faire diminuer la glycémie chez les animaux diabétiques.

Certains composés de formule I présentent aussi un effet insulinosécréteur précoce et de courte durée.

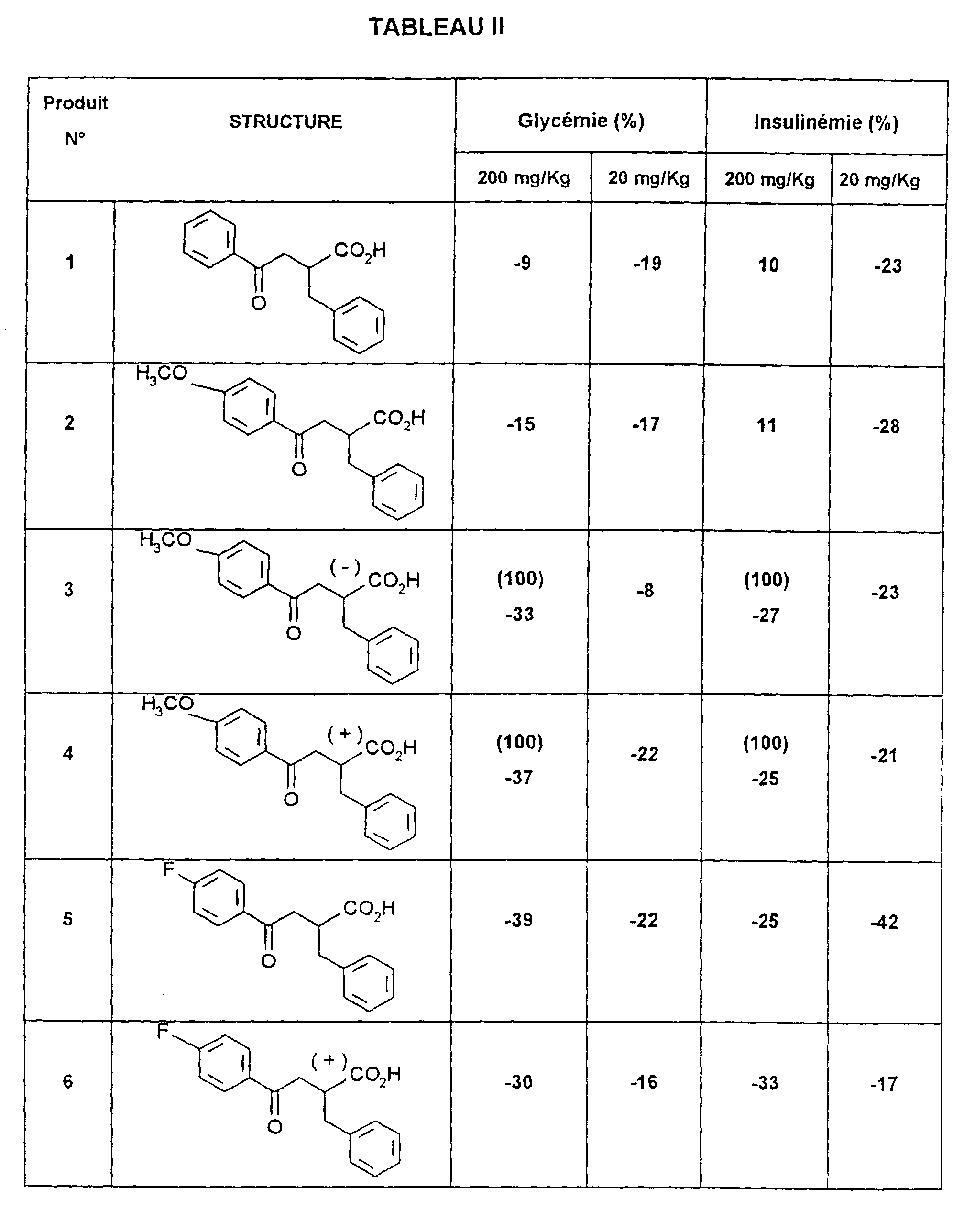

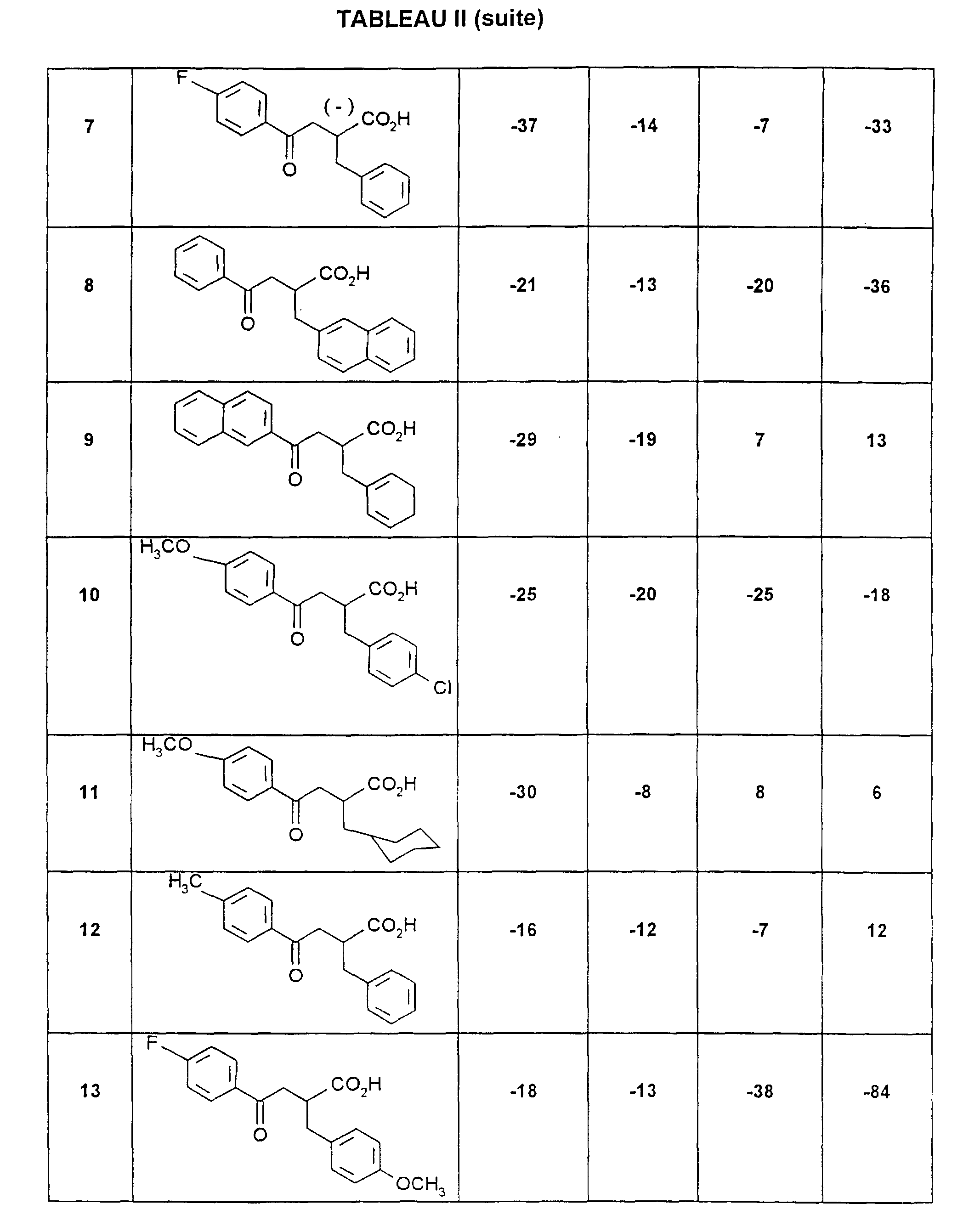

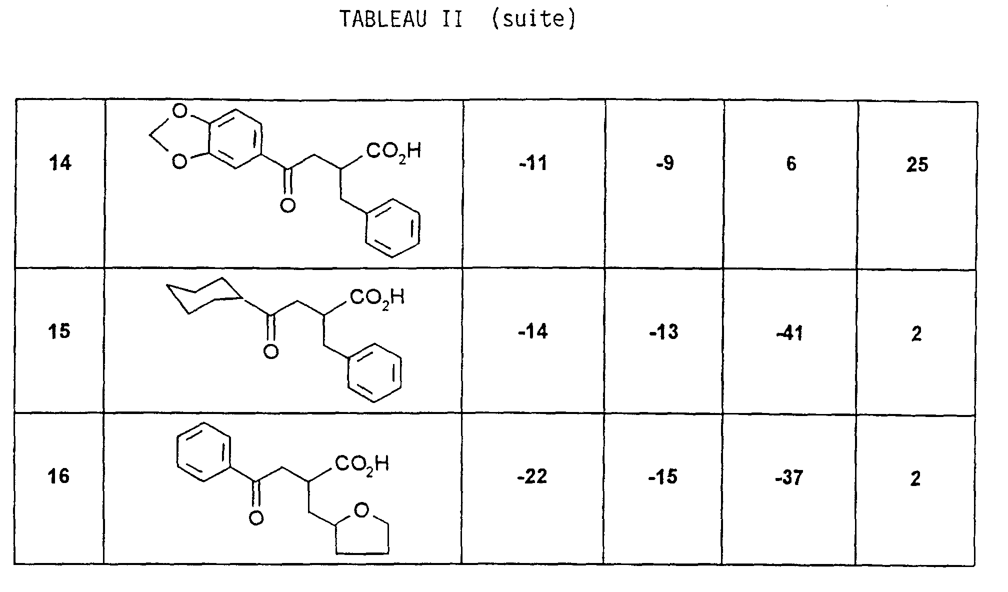

### 2 - Etude chez le rat non diabétique

Le jour de l'expérimentation, des rats non diabétiques sont traités par voie orale avec le produit à tester. Des prélèvements sanguins de 300 *µ*l sont effectués à l'extrémité de la queue des rats pendant les premières 30 minutes qui suivent l'administration du produit.

A titre d'exemple, on donnera les résultats obtenus avec le produit n° 5 (200mg/kg po).

**TABLEAU III**

| Temps après administration (min) | 5 | 10 | 15 | 20 | 30 |
|---|---|---|---|---|---|
| % Glycémie | -4 | -11 | -25 | -31 | -35 |
| % Insulinémie | 102 | 94 | 57 | 55 | 52 |

On observe une baisse de la glycémie, sans observer d'augmentation significative du taux d'insuline mais au contraire une baisse de ce taux.

### 3 - Test de toxicité

Les produits n° 5 et 6 administrés par voie orale à la dose de 200 mg/kg n'ont pas induit de signe de toxicité.

### 4 - Action sur la sécrétion de glucagon

Des expériences in vitro, au niveau de pancréas perfusés de rats non diabétiques isolés selon la technique du Sussman et Coll. *(Diabetes 15 : 466, 1966)* modifiée par Assan et Coll. *(Nature 239 : 125, 1972)* ont montré qu'en l'absence de glucose, dans le milieu de perfusion, ainsi qu'en présence d'arginine, la sécrétion de glucagon était stimulée par les composés de formule I. Dans les mêmes conditions, les sulfonylurées ont un effet inhibiteur marqué. Néanmoins, en présence d'une concentration élevée de glucose, les composés de formule I ne modifient pas l'inhibition de la sécrétion de glucagon par le glucose. Les risques d'hypoglycémies associés au traitement par les sulfonylurées seront ainsi évités lors de traitements par les composés de formule I.

## Revendications

1. Composition pharmaceutique comprenant, à titre de principe actif, un composé de formule : 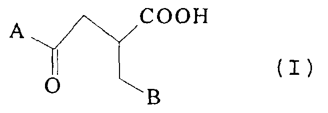
dans laquelle les groupes A et B sont choisis indépendamment l'un de l'autre parmi :
- un groupe aryle mono-, bi- ou tricyclique ayant de 6 à 14 atomes de carbone;
- un groupe hétéroaromatique choisi parmi les groupes pyridyle, pyrimidyle, pyrrolyle, furyle et thiényle;
- un groupe alcoyle ayant de 1 à 14 atomes de carbone;
- un groupe cycloalcoyle ayant de 5 à 8 atomes de carbone;
- un groupe hétérocyclique saturé choisi parmi les groupes tétrahydrofuryle, tétrahydropyrranyle, pipéridinyle et pyrrolidinyle;
les groupes A et B pouvant porter 1 à 3 substituants choisis parmi un groupe alcoyle en C₁-C₆, alcoxy en C₁-C₆, aryle en C₆-C₁₄, hétéroaryle choisi parmi pyridyle, pyrimidyle, pyrrolyle, furyle et thiényle, aryl (C₆-C₁₄) alcoyle (C₁-C₆), aryl (C₆-C₁₄) alcoyl (C₁-C₆) aryle (C₆-C₁₄), un halogène, un groupe trifluorométhyle, trifluorométhoxy, cyano, hydroxy, nitro, amino, carboxy, alcoxy (C₁-C₆)carbonyle, carbamoyle, alcoyl(C₁-C₆)sulfonyle, sulfoamino, alcoyl(C₁-C₆) sulfonylamino, sulfamoyle, alcoyl(C₁-C₆)carbonylamino
ou deux des substituants formant un groupe methylènedioxy, son solvate ou un sel de cet acide avec une base pharmaceutiquement acceptable.

2. Composition pharmaceutique selon la revendication 1 comprenant, à titre de principe actif, un composé de formule I dans laquelle A et B sont choisis parmi des groupes aryle.

3. Compositions selon la revendication 1 comprenant, à titre de principe actif, un composé choisi parmi les composés de formule :
- l'acide 2-benzyl-4-(4-méthoxyphényl)-4-oxobutanoïque
- l'acide 2-benzyl-4-(4-fluorophényl)-4-oxobutanoïque
- l'acide 2-cyclohexylméthyl-4-(4-méthoxyphényl)-4-oxobutanoïque
- l'acide 2-benzyl-4-phényl-4-oxobutanoïque
- l'acide 2-(β-naphthylméthyl)-4-phényl-4-oxobutanoïque
- l'acide 2-benzyl-4-(β-naphtyl)-4-oxobutanoïque
- l'acide 2-[(4-chlorophényl)méthyl]-4-(4-méthoxyphényl)-4-oxobutanoïque
- l'acide 2-benzyl-4-(4-méthylphényl)-4-oxobutanoïque
- l'acide 4-(4-fluorophényl)-2-[(4-méthoxyphényl)méthyl]-4-oxobutanoïque
- l'acide 2-benzyl-4-(3,4-méthylènedioxyphényl)-4-oxobutanoïque
- l'acide 2-benzyl-4-cyclohexyl-4-oxobutanoïque
- l'acide 4-phényl-2-[(tétrahydrofur-2-yl)méthyl]-4-oxobutanoïque,
les solvates et les sels de ces acides avec des bases pharmaceutiquement acceptables.

4. Composés de formule : 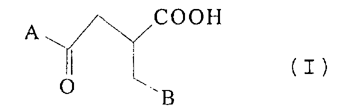
dans laquelle les groupes A et B sont choisis indépendamment l'un de l'autre parmi :
- un groupe aryle mono-, bi- ou tricyclique ayant de 6 à 14 atomes de carbone;
- un groupe hétéroaromatique choisi parmi les groupes pyridyle, pyrimidyle, pyrrolyle, furyle et thiényle;
- un groupe alcoyle ayant de 1 à 14 atomes de carbone;
- un groupe cycloalcoyle ayant de 5 à 8 atomes de carbone;
- un groupe hétérocyclique saturé choisi parmi les groupes tétrahydrofuryle, tétrahydropyrranyle, pipéridinyle et pyrrolidinyle;
les groupes A et B pouvant porter 1 à 3 substituants choisis parmi un groupe alcoyle en C₁-C₆, alcoxy en C₁-C₆, aryle en C₆-C₁₄, hétéroaryle choisi parmi pyridyle, pyrimidyle, pyrrolyle, furyle et thiényle, aryl (C₆-C₁₄) alcoyle (C₁-C₆), aryl (C₆-C₁₄) alcoyl (C₁-C₆) aryle (C₆-C₁₄), un halogène, un groupe trifluorométhyle, trifluorométhoxy, cyano, hydroxy, nitro, amino, carboxy, alcoxy (C₁-C₆)carbonyle, carbamoyle, alcoyl(C₁-C₆)sulfonyle, sulfoamino, alcoyl(C₁-C₆) sulfonylamino, sulfamoyle, alcoyl(C₁-C₆)carbonylamino
ou deux des substituants formant un groupe methylènedioxy,
à l'exclusion des composés de formule I dans laquelle B est un groupe phényle non substitué et A est un groupe phényle, 4-méthoxyphényle, 4-chlorophényle ou cyclohexyle,
leurs solvates et les sels de ces acides avec des bases.

## Patentansprüche

1. Pharmazeutische Zusammensetzung, umfassend als aktiven Wirkstoff eine Verbindung der Formel
wobei die Gruppen A und B unabhängig voneinander ausgewählt werden aus:
- einer mono-, bi- oder tricyclischen Arylgruppe mit 6 bis 14 Kohlenstoffatomen;
- einer heteroaromatischen Gruppe, die ausgewählt wird aus den Gruppen Pyridyl, Pyrimidyl, Pyrrolyl, Furyl und Thienyl,
- einer Alkylgruppe mit 1 bis 14 Kohlenstoffatomen;
- einer Cycloalkylgruppe mit 5 bis 8 Kohlenstoffatomen;
- einer gesättigten heterocyclischen Gruppe, die ausgewählt wird aus den Gruppen Tetrahydrofuryl, Tetrahydropyranyl, Piperidinyl and Pyrrolidinyl:
wobei die Gruppen A und B 1 bis 3 Substituenten aufweisen können, die ausgewählt werden aus einer C₁-C₆ Alkylgruppe, C₁-C₆ Alkoxygruppe, C₆-C₁₄ Arylgruppe, Heteroarylgruppe, die ausgewählt wird aus Pyridyl, Pyrimidyl, Pyrrolyl, Furyl und Thienyl, (C₆-C₁₄)Aryl(C₁-C₆)alkyl, (C₆-C₁₄)Aryl(C₁-C₆)alkyl(C₆-C₁₄)aryl, Halogen, Trifluoromethyl-, Trifluoromethoxy-, Cyano-, Hydroxy-, Nitro-, Amino-, Carboxy-, (C₁-C₆)Alkoxycarbonyl-, Carbamoyl-, (C₁-C₆)Alkylsulfonyl-, Sulfoamino-, (C₁-C₆)Alkylsulfonylamino-, Sulfamoyl-, (C₁-C₆)Alkylaminogruppe,
oder zwei Substituenten eine Methylendioxygruppe bilden, ihr Solvat oder ein Salz dieser Säure mit einer pharmazeutisch verträglichen Base.

2. Pharmazeutische Zusammensetzung nach Anspruch 1, umfassend als aktiven Wirkstoff eine Verbindung der Formel I, wobei A und B aus Arylgruppen ausgewählt werden.

3. Zusammensetzung nach Anspruch 1, umfassend als aktiven Wirkstoff eine Verbindung, die ausgewählt wird aus den Verbindungen der Formel:
- 2-Benzyl-4-(4-methoxyphenyl)-4-oxobutansäure
- 2-Benzyl-4-(4-fluorophenyl)-4-oxobutansäure
- 2-Cyclohexylmethyl-4-(4-methoxyphenyl)-oxobutansäure
- 2-Benzyl-4-phenyl-4-oxobutansäure
- 2-(ß-Naphthylmethyl)-4-phenyl-4-oxobutansäure
- 2-Benzyl-4-(ß-naphtyl)-4-oxobutansäure
- 2-[(4-Chlorophenyl)methyl]-4-(4-methoxyphenyl)-4-oxobutansäure
- 2-Benzyl-4-(4-methylphenyl)-4-oxobutansäure
- 4-(4-Fluorophenyl)-2-[(4-methoxyphenyl)methyl]-4-oxobutansäure
- 2-Benzyl-4-(3,4-methylendioxyphenyl)-4-oxobutansäure
- 2-Benzyl-4-cyclohexyl-4-oxobutansäure
- 4-Phenyl-2-[(tetrahydrofur-2-yl)methyl]-4-oxobutansäure,
ihre Solvate und die Salze dieser Säuren mit pharmazeutisch verträglichen Basen.

4. Verbindungen der Formel
wobei die Gruppen A und B unabhängig voneinander ausgewählt werden aus:
- einer mono-, bi- oder tricyclischen Arylgruppe mit 6 - 14 Kohlenstoffatomen;
- einer heteroaromatischen Gruppe, die ausgewählt wird aus den Gruppen Pyridyl, Pyrimidyl, Pyrrolyl, Furyl und Thienyl,
- einer Alkylgruppe mit 1 - 14 Kohlenstoffatomen;
- einer Cycloalkylgruppe mit 5 - 8 Kohlenstoffatomen;
- einer gesättigten heterocyclischen Gruppe, die ausgewählt wird aus den Gruppen Tetrahydrofüryl, Tetrahydropyranyl, Piperidinyl und Pyrrolidinyl;
wobei die Gruppen A und B 1 bis 3 Substituenten aufweisen können, die ausgewählt werden aus einer C₁-C₆ Alkylgruppe, C₁-C₆ Alkoxygruppe, C₆-C₁₄ Arylgruppe, Heteroarylgruppe, die ausgewählt wird aus Pyridyl, Pyrimidyl, Pyrrolyl, Furyl und Thienyl, (C₆-C₁₄)Aryl(C₁-C₆)alkyl, (C₆-C₁₄)Aryl(C₁-C₆)alkyl(C₆-C₁₄)aryl, Halogen, Trifluoromethyl-, Trifluoromethoxy-, Cyano-, Hydroxy-, Nitro-, Amino-, Carboxy-, (C₁-C₆)Alkoxycarbonyl-, Carbamoyl-, (C₁-C₆)Alkylsulfonyl-, Sulfoamino-, (C₁-C₆)Alkylsulfonylamino-, Sulfamoyl-, (C₁-C₆)Alkylaminogruppe,
oder zwei Substituenten eine Methylendioxygruppe bilden,
unter Ausschluß von Verbindungen der Formel I, bei denen B eine unsubstituierte Phenylgruppe ist und A eine Phenyl-, 4-Methoxyphenyl-, 4-Chlorophenyl oder Cyclohexylgruppe ist,
ihre Solvate und die Salze dieser Säure mit Basen.

## Claims

1. Pharmaceutical composition comprising, as active ingredient, a compound of formula: 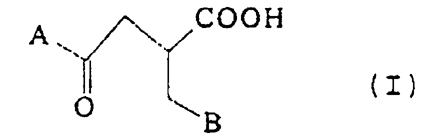
in which the groups A and B are chosen, independently of each other, from:
- a mono-, bi- or tricyclic aryl group having from 6 to 14 carbon atoms;
- a heteroaromatic group chosen from pyridyl, pyrimidyl, pyrrolyl, furyl and thienyl groups;
- an alkyl group having from 1 to 14 carbon atoms;
- a cycloalkyl group having from 5 to 8 carbon atoms;
- a saturated heterocyclic group chosen from tetrahydrofuryl, tetrahydropyranyl, piperidinyl and pyrrolidinyl groups;
it being possible for the groups A and B to carry 1 to 3 substituents chosen from a C₁-C₆ alkyl group, a C₁-C₆ alkoxy group, a C₆-C₁₄ aryl group, a heteroaryl group chosen from pyridyl, pyrimidyl, pyrrolyl, furyl and thienyl, a (C₆₋C₁₄)aryl(C₁-C₆)alkyl group, a (C₆-C₁₄)aryl(C₁-C₆)alkyl(C₆-C₁₄)aryl group, a halogen, a trifluoromethyl, trifluoromethoxy, cyano, hydroxyl, nitro, amino, carboxyl, (C₁-C₆)alkoxycarbonyl, carbamoyl, (C₁-C₆)alkylsulfonyl, sulfoamino, (C₁-C₆)alkylsulfonylamino, sulfamoyl, (C₁-C₆)alkylcarbonylamino group
or two of the substituents forming a methylenedioxy group, its solvate or a salt of this acid with a pharmaceutically acceptable base.

2. Pharmaceutical composition according to Claim 1 comprising, as active ingredient, a compound of formula I in which A and B are chosen from aryl groups.

3. Compositions according to Claim 1 comprising, as active ingredient, a compound chosen from the compounds of formula:
- 2-benzyl-4-(4-methoxyphenyl)-4-oxobutanoic acid
- 2-benzyl-4-(4-fluorophenyl)-4-oxobutanoic acid
- 2-cyclohexylmethyl-4-(4-methoxyphenyl)-4-oxobutanoic acid
- 2-benzyl-4-phenyl-4-oxobutanoic acid
- 2-(β-naphthylmethyl)-4-phenyl-4-oxobutanoic acid
- 2-benzyl-4-(β-naphthyl)-4-oxobutanoic acid
- 2-[(4-chlorophenyl)methyl]-4-(4-methoxyphenyl)-4-oxobutanoic acid
- 2-benzyl-4-(4-methylphenyl)-4-oxobutanoic acid
- 4-(4-fluorophenyl)-2-[(4-methoxyphenyl)methyl]-4-oxobutanoic acid
- 2-benzyl-4-(3,4-methylenedioxyphenyl)-4-oxobutanoic acid
- 2-benzyl-4-cyclohexyl-4-oxobutanoic acid
- 4-phenyl-2-[(tetrahydrofur-2-yl)methyl]-4-oxobutanoic acid,
the solvates and the salts of these acids with pharmaceutically acceptable bases.

4. Compounds of formula: 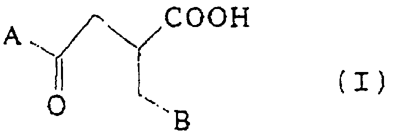
in which the groups A and B are chosen, independently of each other, from:
- a mono-, bi- or tricyclic aryl group having from 6 to 14 carbon atoms;
- a heteroaromatic group chosen from pyridyl, pyrimidyl, pyrrolyl, furyl and thienyl groups;
- an alkyl group having from 1 to 14 carbon atoms;
- a cycloalkyl group having from 5 to 8 carbon atoms;
- a saturated heterocyclic group chosen from tetrahydrofuryl, tetrahydropyranyl, piperidinyl and pyrrolidinyl groups;
it being possible for the groups A and B to carry 1 to 3 substituents chosen from a C₁-C₆ alkyl group, a C₁-C₆ alkoxy group, a C₆-C₁₄ aryl group, a heteroaryl group chosen from pyridyl, pyrimidyl, pyrrolyl, furyl and thienyl, a (C₆-C₁₄)aryl(C₁-C₆)alkyl group, a (C₆-C₁₄)aryl(C₁-C₆)alkyl(C₆-C₁₄)aryl group, a halogen, a trifluoromethyl, trifluoromethoxy, cyano, hydroxyl, nitro, amino, carboxyl, (C₁-C₆)alkoxycarbonyl, carbamoyl, (C₁-C₆)alkylsulfonyl, sulfoamino, (C₁-C₆)alkylsulfonylamino, sulfamoyl, (C₁-C₆)alkylcarbonylamino group
or two of the substituents forming a methylenedioxy group,
excluding the compounds of formula I in which B is an unsubstituted phenyl group and A is a phenyl, 4-methoxyphenyl, 4-chlorophenyl or cyclohexyl group,
their solvates and the salts of these acids with bases.
